# EUROPEAN PATENT APPLICATION

(11) **EP 2 589 333 A1**
(43) Date of publication of application: **08.05.2013**
(21) Application number: 12184254.6
(22) Date of filing: 13.09.2012
(51) Int. Cl.: A61B 5/0432, A61B 5/0452, A61B 5/0472, A61B 5/0408

(54) **Apparatus and system for long-term cutaneous cardiac monitoring**

(30) Priority: 04.11.2011 US 201161555512 P
(71) Applicant: BIOTRONIK SE & Co. KG, 12359 Berlin (DE)
(72) Inventor: Lian, Jie, Beaverton, OR 97007 (US); Müssig, Dirk, West Linn, 97068 (US); Lang, Volker, 12161 Berlin (DE)
(74) Representative: Lindner-Vogt, Karin L.

(57) **Abstract**

The invention refers to a system comprising:
(1) a disposable unit that has at least two built-in electrodes, a built-in wire antenna, a watertight chamber that can be opened and closed, and an adhesive surface;
(2) an electronic controller that can acquire, process and store the physiological signals and can be fitted into the disposable unit and establish the electrical contact with the at least two built-in electrodes; and
(3) a portable communication unit that can wirelessly communicate bi-directionally with the electronic controller and further communicate bi-directionally with a remote service center.

## Description

The invention relates to an apparatus and a system that is suitable for long-term non-invasive heart monitoring.

Conventionally, Holter monitors have been used for non-invasive monitoring of heart rhythm, but the monitoring time is usually limited to 24-48 hours, at most 7 days.

Event recorders and external loop recorders have also been used for ambulatory cardiac monitoring, usually lasting a week or up to 30 days. These devices can be programmed to record short episodes of ECG that are manually triggered by the patient upon experiencing symptoms, or automatically record a limited number of arrhythmia episodes detected by the device.

A long-term continuous ECG recorder (Zio™ patch) has also been developed by iRhythm. This disposable device can be worn by the patient for up to two weeks and continuously record ECG. After finishing the recording, the device is mailed to the service center which downloads the recorded ECG and performs offline ECG analysis.

A wireless ECG monitoring system is disclosed in US 2011/0125040 A1 that includes a disposable patch having electrodes and an adhesive surface for attachment to the chest of the patient, an ECG monitor that can be hooked up with the disposable patch, and a cell phone handset that can bi-directionally communicate with the ECG monitor. The system is designed to continuously monitor ECG for at least 24 hours and repeat the ECG monitoring after recharging the battery and using new disposable patches.

Implantable loop recorder has been used for diagnosis of unexplained syncope and detection of atrial fibrillation. The device is suitable for long-term monitoring (usually >1 year).

Holter monitors, wearable event recorders and external loop recorders are not suitable for long term monitoring (maximum 1 mo). The electrodes usually require daily change and the wires are cumbersome.

Although Zio™ patch is waterproof and can be worn comfortably by a patient for up to one or two weeks, it is also not designed for long-term monitoring (e.g. >6 mo).

The wireless ECG monitoring system disclosed in US 2011/0125040 Al has two major limitations. First, the need of periodically recharging the ECG monitor makes it impossible for continuous cardiac monitoring. When the ECG monitor is removed from the patch and recharged (for example when the patient is sleeping), no cardiac monitoring can be performed. Second, when switching from one disposable patch to another, there is no quantitative guidance for the patient to properly place the patch over the chest surface. The patient has to rely on a printed guide to find the location and adjust the orientation of the patch, but there is no feedback to the patient whether the placement of the patch is appropriate. As a result, the recorded ECG, after switching the disposable patches, may have low quality and cannot be used, or the newly acquired ECG may not be compared to previous recordings.

Implantable loop recorders are the only devices currently available for long-term cardiac monitoring, but it requires invasive operation for implanting the device under the skin.

It is an object of the invention to provide an improved apparatus and system for long-term non-invasive monitoring of cardiac functions.

According to the invention, this object is achieved by a system comprising:
(1) a disposable unit that has at least two built-in electrodes, a built-in wire antenna, a watertight chamber that can be opened and closed, and an adhesive surface;
(2) an electronic controller that can acquire, process and store the physiological signals, and can be fitted into the disposable unit and establish the electrical contact with the at least two built-in electrodes and the built-in wire antenna; and
(3) a portable communication unit that can wirelessly communicate bi-directionally with the electronic controller and further communicate bi-directionally with a remote service center. The electronic controller can also communicate bi-directionally with another programming device.

Further, the object of the invention is achieved by an apparatus for long-term cutaneous cardiac monitoring: comprising a disposable unit configured to be adhered to a patient's skin, and an electronic controller detachably connected to the disposable unit. The disposable unit comprises
- a substantially flat and flexible skin contacting part that has at least two pick-up electrode poles arranged to electrically contact a patient's skin at at least two spaced-apart locations,
- a wire antenna; and
- a watertight chamber attached to or integrated within the flat and flexible skin contacting part, and being configured to accommodate the electronic controller in a watertight sealed manner and being able to open and close so as to allow removal and reinsertion of the electronic controller. On the inside of the chamber, electrical terminals are provided that are electrically connected to the at least two pick-up electrode poles and that are arranged to be operatively connected to respective ports of the electronic controller. Likewise, a conducting antenna port is provided inside the chamber. The conducting antenna port is electrically connected to the wire antenna and arranged to be operatively connected to a respective antenna port of the electronic controller.

The electronic controller comprises a battery with a capacity sufficient to allow the electronic controller to operate for at least one year. The electronic controller has contact ports arranged to make contact to the terminals within the chamber and to thus establish electrical contact with the pick-up electrode poles and the wire antenna, respectively.

The electronic controller is configured to acquire, process, and store the physiological signals to be picked up via the at least two electrode poles and to provide quantitative feedback on signal quality of a signal acquired via the at least two pick-up electrode poles.

The proposed system thus has 3 parts: (1) a disposable unit that has at least two built-in electrodes, a built-in wire antenna, a waterproof chamber that can be opened and closed, and an adhesive surface; (2) an electronic controller that has a battery life of at least 1 year and can acquire, process and store the physiological signals and can be fitted into the disposable unit and establish the electrical contact with the at least two built-in electrodes and the built-in wire antenna; and (3) a portable communication unit that can wirelessly communicate bi-directionally with the electronic controller and further communicate bi-directionally with a remote service center. The electronic controller can also communicate bi-directionally with another programming device. Two of these arts, namely the disposable unit and the electronic controller, are physically connected to each other when in use and form a body-worn monitoring apparatus.

One unique feature of this system is that the disposable unit, the electronic controller and the portable communication unit are physically decoupled but functionally coupled. The long-term non-invasive cardiac monitoring is achieved by connecting these components electrically (between the disposable unit and the electronic controller) and wirelessly (between the electronic controller and the portable communication unit).

The disposable unit can be comfortably worn by the patient for 1-2 weeks. The patient can fit the electronic controller into the disposable unit to complete the circuit for ECG monitoring. The electronic controller also connects to the wire antenna in the disposable patch for wireless communication with the portable communication unit.

The patient can remove the electronic controller from one disposable unit and place it in another disposable unit to continue cardiac monitoring. Upon switching to a new disposable unit, the patient can use the portable communication unit to interrogate the electronic controller to check the quality of the acquired ECG signal. Quantitative feedback on the ECG signal quality, such as the morphological similarity between acquired ECG waveform and the pre-stored ECG templates as well as the signal-to-noise ratio can be provided to the patient to guide the proper placement of the disposable unit on the body surface.

The ECG and other diagnostic data stored in the electronic controller can periodically (or event-triggered) be transmitted to the external portable communication unit which relays the information to a remote service center. After data transmission, the electronic controller preferably clears the data memory and continues heart monitoring. The battery of the electronic controller supports the normal operation of the electronic controller for at least 12 months. Therefore, by changing the disposable unit every 1-2 weeks, the system can continuously monitor the cardiac rhythm for at least 12 months.

The electronic controller continuously monitors the skin contact of the electrodes in the disposable unit and communicates the related information to the external portable communication unit which provides instantaneous feedback for the patient. The electronic controller can alert the patient to reposition or replace the disposable unit upon receiving a warning signal from the electronic controller indicating abnormal skin-electrode contact, or based on pre-configured schedule.

According to a preferred embodiment, the electronic controller is further configured to provide quantitative feedback on signal quality of a signal acquired via the at least two pick-up electrode poles. Further, the electronic controller can be configured to maintain at least one normal ECG beat template. In particular, the electronic controller is further configured to construct the normal ECG beat template by averaging multiple normal beats of ECG signals acquired via the pick-up electrode poles and being aligned with predefined fiducial points. Even more particular, the electronic controller is configured to start constructing the normal ECG beat template immediately upon affixing the disposable unit on a patient's skin, and to continuously update the normal ECG beat template based on acquired ECG signals.

The disposable unit preferably has three built-in electrode poles. Preferably, these electrode poles are spaced apart from each other by an inter-electrode distance of at least 3 cm.

The electronic controller preferably comprises electronic and electric components that are hermetically sealed inside a case of the electronic controller. These electric and electronic components preferably include an electronic circuitry including a microprocessor, a memory and an electronic interface circuitry preferably including a feedthrough circuitry for noise reduction, a high voltage protection circuitry, a switch network circuitry for sensing channel selection, and front-end analog filters, and an ECG sensing unit connected to the electronic interface circuitry and the microcontroller, the ECG sensing unit comprising amplifiers, analog-to-digital converters, digital filters, etc.

According to a further preferred embodiment, the electronic controller comprises an impedance measuring unit that connects to the conducting ports for connecting the terminals, the impedance measuring unit being configured to measure an impedance signal between the at least two electrode poles. Thus, in the preferred embodiment, the electronic controller can do both, picking up ECG signals and measuring inter-electrode impedance.

Regarding the system, it is preferred that the portable communication unit is configured to interrogate the electronic controller to check the morphology and quality of an acquired ECG signal. In particular, it is preferred that the system is configured to wirelessly transmit a latest normal ECG beat template from the electronic controller to the portable communication unit and then compare a newly acquired normal ECG beat template with an old normal ECG beat templates that are pre-stored in the portable communication unit.

In the system, preferably the portable communication unit and/or the electronic controller is configured to rate a newly acquired normal ECG beat template acceptable only when its morphology is sufficiently similar to at least one of the old normal ECG beat templates stored in the portable communication unit. Alternatively or additionally, the portable communication unit and/or the electronic controller can be configured to rate a newly acquired normal ECG beat template acceptable only when it has sufficiently high signal quality, in particular, if the signal-to-noise ratio of an QRS complex in the ECG beat template is greater than a predefined threshold. Alternatively or additionally, the portable communication unit and/or the electronic controller can be configured to rate a newly acquired normal ECG beat template acceptable only when its morphology is sufficiently similar to at least one of the old normal ECG beat templates and it has sufficiently high signal quality as measured by the signal to noise ratio.

According to a further preferred embodiment, the portable communication unit and/or the electronic controller is configured to generate a signal indicating a necessity to reposition the disposable unit if the newly acquired normal ECG beat template is rated not acceptable. Thus, the system can assist a patient in positioning the disposable unit in case it has to be replaced. Furthermore, consistency between ECG signals being picked up prior to replacement with ECG-signals being picked up after replacement is maintained.

The above and other aspects, features and advantages of the present invention will be more apparent from the following more particular description thereof, presented in conjunction with the following drawings wherein:
- FIG. 1: shows (A) the bottom view and (B) the side view of the disposable unit 100 and the electronic controller 200;
- FIG. 2: illustrates that the disposable unit 100 has a cover 160 over the top (Figure 2A) or the bottom (Figure 2B);
- FIG. 3: illustrates (A) the bottom view and (B) the side view of the disposable unit 100 with its chamber 150 housing the electronic controller 200;
- FIG. 4: shows (A) the bottom view and (B) the side view of the disposable unit 100 and the corresponding electronic controller 200 ;
- FIG. 5: schematically illustrates the preferred operation of the long-term cardiac monitoring system;
- FIG. 6: illustrates that the portable communication unit 600 typically stores multiple (preferably at least 3) old normal ECG beat templates;
- FIG. 7: shows a block diagram of an electronic controller 200 connecting with a disposable unit 100 for cutaneous ECG monitoring and its interfaces with an external programming device 800 and a portable communication unit 600 which further communicates with the remote service center 700;
- FIG. 8: shows that the external portable communication unit 600 has a button 610 which can be pressed by the patient to trigger an event recording by the electronic controller 200

The following description is of the best mode presently contemplated for carrying out the invention. This description is not to be taken in a limiting sense but is made merely for the purpose of describing the general principles of the invention. The scope of the invention should be determined with reference to the claims.

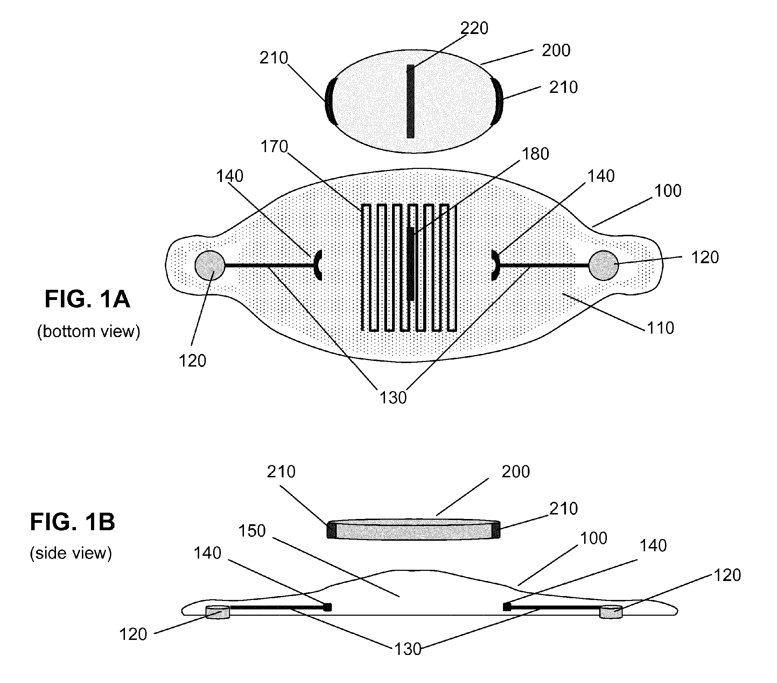

Figure 1 shows (A) the bottom view and (B) the side view of the disposable unit 100 and the electronic controller 200.

To achieve long-term non-invasive cardiac monitoring, a lightweight, water-proof and disposable skin-adhesive unit 100 is provided. Because the unit is disposable, it can be easily replaced with another disposable unit after its usage for a few days. The bottom of the disposable unit 100 has an adhesive surface 110 that can be affixed to the patient's skin for at least 7 days. One exemplary type of such adhesive material is the pressure-sensitive adhesive which forms a bond when pressure is applied to stick the adhesive to the adherent.

The disposable unit 100 has at least two built-in electrodes 120. Preferably, the inter-electrode distance is greater than 3 cm. The electrodes 120 are embedded inside the disposable unit 100 but protrude from its bottom surface so that they can establish contact with the patient's skin. The electrodes 120 are connected to the respective electrical conducting terminals 140 via the conducting wires 130. Both conducting wires 130 and the electrical conducting terminals 140 are enclosed inside the disposable unit 100 which has a watertight housing that is made of a flexible biocompatible polymer.

Preferably, the disposable unit 100 also has a built-in wire antenna 170. Because of the relatively large surface area of the disposable unit, a long wire antenna 170 (e.g. >6 cm in length) can be embedded, for example by arranging the wire antenna 170 in a zigzag pattern or a multiple loop pattern. The wire antenna 170 also has a conducting port 180. Both, the wire antenna 170 and its conducting port 180, are enclosed inside the disposable unit 100.

The electronic controller 200 comprises an electronic circuitry that is hermetically sealed inside a case which is preferably made from a biocompatible material. At least two electrical conducting ports 210, which are isolated from each other, are embedded on the case of the electronic controller 200. In addition, a separate conducting antenna interface 220 is also embedded on the case of the electronic controller 200. In normal working condition as described thereinafter, the electronic controller 200 has a battery life of at least 1 year without the need of recharging.

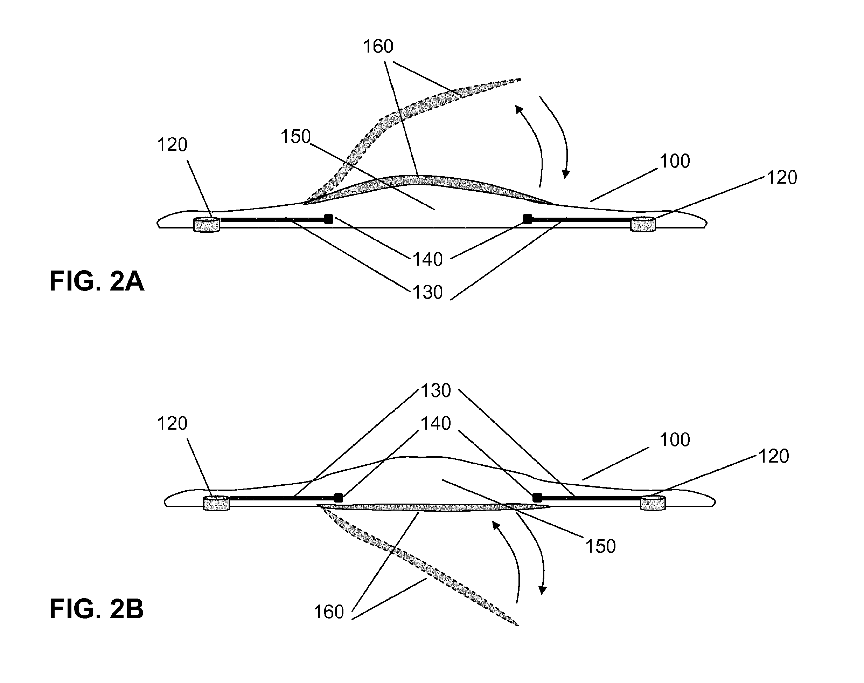

The disposable unit 100 has a hollow chamber 150, the size of which matches the size of the electronic controller 200. As illustrated in Figure 2, the disposable unit 100 has a cover 160 over the top (Figure 2A) or the bottom (Figure 2B). The cover 160 is made of a flexible biocompatible polymer and can be opened to expose the chamber 150 or closed and tightly seal the chamber 150.

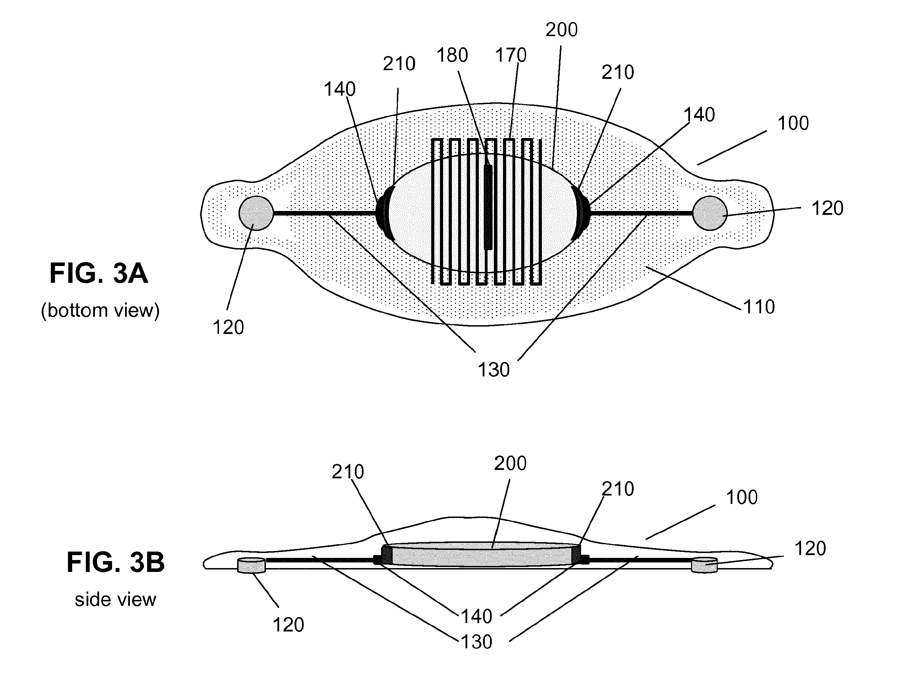

Therefore, the electronic controller 200 can be conveniently placed inside or removed from the chamber 150 of the disposable unit 100. Figure 3 illustrates (A) the bottom view and (B) the side view of the disposable unit 100 with its chamber 150 housing the electronic controller 200. Mechanical design is made so that the electronic controller 200 is secured in its place after it is snug fit inside the chamber 150. The electrical conducting ports 210 on the surface of the electronic controller 200 make secure contact with the respective electrical conducting terminals 140 embedded inside the disposable unit 100, so that they are electrically connected to the respective electrodes 120. In addition, the antenna interface 220 on the surface of the electronic controller 200 makes secure contact with the conducting port 180 of the wire antenna 170 embedded inside the disposable unit 100, so that the electronic controller 200 electrically connects with the wire antenna 170.

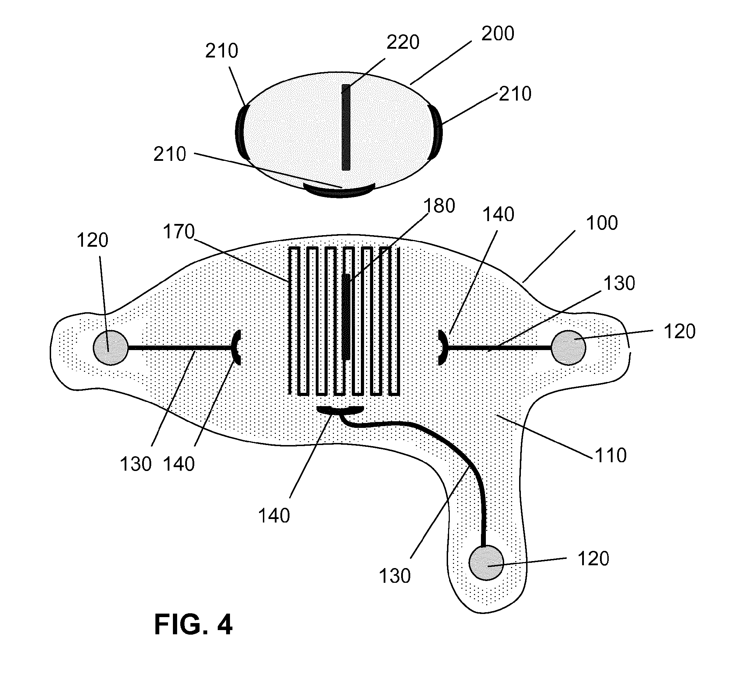

The above design can be easily extended to multi-electrode configuration. For example, Figure 4 shows (A) the bottom view and (B) the side view of the disposable unit 100 which has three built-in electrodes 210, a built-in wire antenna 170 that has a conducting port 180, and the corresponding electronic controller 200 which has three electrical conducting ports 210 on its surface and an antenna interface 220. The geometric shape of the disposable unit 100 and the topographic distribution of the built-in electrodes 120 can be predefined or custom-designed to fit different torso shapes of the patients and to optimize the ECG recordings (for example, selection of different ECG lead vectors, increase the signal-to-noise ratio of ECG signals, etc.).

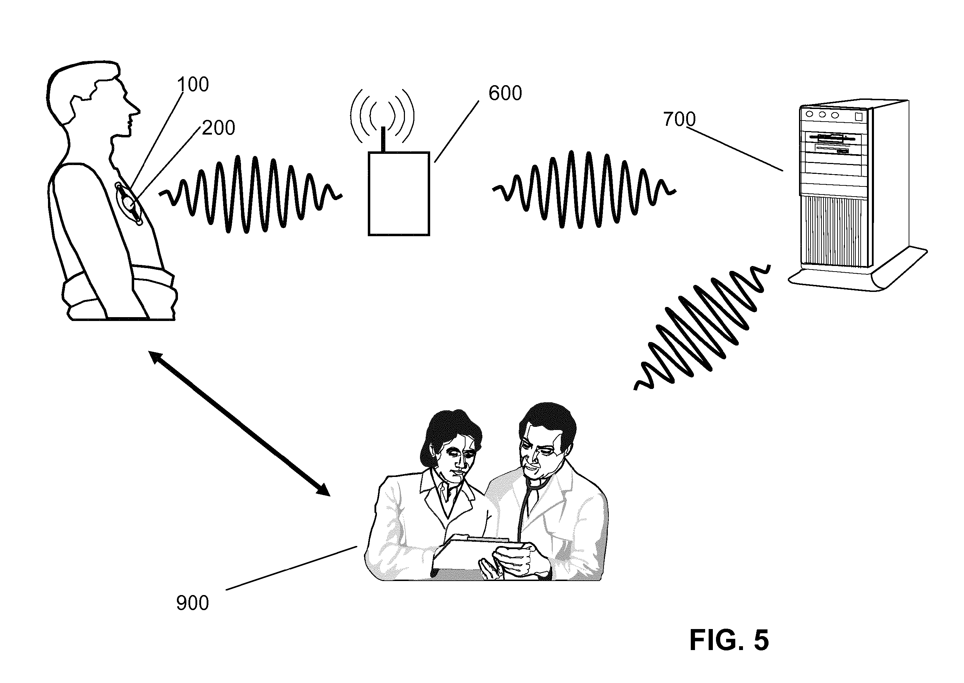

Figure 5 schematically illustrates the preferred operation of the long-term cardiac monitoring system. The patient fits the electronic controller 200 into the disposable unit 100 which is affixed to the patient's chest, preferably over the heart. The electronic controller 200 continuously monitors the impedance between its electrical conducting ports 210 which are connected to the electrodes 120 after fitting the electronic controller 200 into the disposable unit 100. The impedance value is high (usually >1MΩ) when the electrodes 120 are isolated from each other. When the disposable unit 100 is affixed to the patient's skin, the body tissue forms an electrical conductor. Thus, the electronic controller 200 can immediately detect the drop of the impedance value to the predefined normal range and then automatically start self-initialization process for cutaneous ECG monitoring.

The electronic controller 200 acquires the cutaneous ECG signal measured between the at least two electrodes 120 embedded in the disposable unit 100, analyzes the said cutaneous ECG signal and automatically triggers recording of the ECG episode upon detection of abnormal cardiac rhythm. Besides, patient can manually trigger the recording of ECG episode by pressing a button or switch on the external portable communication unit 600, which then sends a command to the electronic controller 200 to initiate the episode recording. Alternatively, the manual trigger for the episode recording can be placed on the disposable unit 100 or the outer surface of the electronic controller 200.

The external portable communication unit 600 can establish and maintain bi-directional wireless communications with the electronic controller 200. Through this communication link, the external portable communication unit 600 can both send commands to and retrieve diagnostic information from the electronic controller 200. The external portable communication unit 600 also can establish and maintain bi-directional communications with the remote service center 700 through wired or wireless Home Monitoring network, through which the diagnostic information retrieved from the electronic controller 200 can be relayed to the remote service center 700 and reviewed by the patient's health care providers 900.

According to a typical embodiment, the cutaneous ECG episodes and related diagnostic information recorded by the electronic controller 200 are periodically transmitted to the remote service center 700 (e.g. every midnight). In another typical embodiment, the recorded ECG episodes and related diagnostic information are immediately transmitted to the remote service center 700 upon patient trigger or automatic detection of predefined severe cardiac conditions (e.g. long-lasting atrial fibrillation, very high or very low ventricular rate, etc.). Yet in another embodiment, the recorded data are transmitted to the remote service center 700 when the memory buffer of the electronic controller 200 is filled to a predefined percentage of its capacity (e.g. 80%). Preferably, after data transmission, the electronic controller 200 clears the data memory so that no data is lost due to memory saturation and continues ECG monitoring and data logging thereinafter.

During ambulatory cardiac monitoring, the electronic controller 200 continues to monitor the impedance between the electrodes 120 as well as the signal quality of the cutaneous ECG. When the electronic controller 200 detects the impedance value falls outside the predefined normal range, it generates a warning signal which is sent to the external portable communication unit 600 which then alerts the patient via different means (e.g. visual, audio, vibration, etc.). In addition, the electronic controller 200 also continuously evaluates the signal quality of the acquired cutaneous ECG signal. If the signal-to-noise ratio (SNR) of the ECG signal has significantly degraded, then a warning signal can also be generated and the patient is alerted.

The patient alert function is critically important for long-term cutaneous cardiac monitoring. For example, loose electrode-skin contact can cause higher than normal impedance, and sweat or water may partially short circuit the electrodes and cause lower than normal impedance. After receiving the automatically generated alert of abnormal impedance or low SNR, patient can reposition the disposable unit 100 over the chest until the impedance value between electrodes is within normal range and the cutaneous ECG has sufficiently high SNR.

If the disposable unit 100 has been worn out after a few days, the patient can choose to remove the electronic controller 200 from the old disposable unit and place it in a new disposable unit to continue cardiac monitoring. Patient can be reminded to change the disposable unit 100 when the electronic controller 200 detects abnormal impedance between electrodes or low SNR of the acquired ECG and generates an alert through the external portable communication unit 600. Alternatively, the external portable communication unit 600 can be configured by the patient to set periodic alarm (e.g. every 7 days) as a reminder for changing the disposable unit 100. Therefore, the automatic alert feature can greatly improve the patient's compliance of regularly changing disposable unit 100, which is essential for long-term cutaneous cardiac monitoring.

For the purpose of long-term cardiac monitoring, it is desirable that the recording ECG lead vectors remain stable, or at least they should be well-defined, so that appropriate interpretation of ECG can be made. This requirement may pose a special challenge when patient changes the disposable unit 100. For example, patient may choose to apply the new disposable unit 100 to a different chest location than the old one to prevent skin irritation from repeated use. In another example, patient may intend to replace the disposable unit 100 at the same chest location, but the actual location and/or orientation of the new disposable unit 100 may still differ from the previous one because of human error, even if the patient is aided with a graphical guide.

According to this invention, quantitative feedback is provided to the patient to guide the proper replacement of the disposable units as described in details hereinafter.

According to a typical embodiment of the invention, the electronic controller 200 maintains a normal ECG beat template. As known in the art, the normal ECG beat template can be obtained by averaging multiple (e.g. 8, 16, etc.) normal beats of ECG signals aligned with predefined fiducial point (e.g. the peak of QRS complex). Abnormal beats (such as ectopic beats or cardiac cycles during cardiac arrhythmia) are automatically detected and excluded from beat averaging. The electronic controller 200 starts constructing the normal ECG beat template immediately upon affixing the disposable unit on the patient's skin and continuously updates the normal ECG beat template based on the acquired ECG signal.

In a preferred embodiment, after switching to a new disposable unit 100, patient can use the portable communication unit 600 to interrogate the electronic controller 200 to check the morphology and quality of the acquired ECG signal. Typically, the latest normal ECG beat template (at the time of interrogation) is wirelessly transmitted from the electronic controller 200 to the portable communication unit 600. The newly acquired normal ECG beat template is then compared to the old normal ECG beat templates pre-stored in the portable communication unit 600.

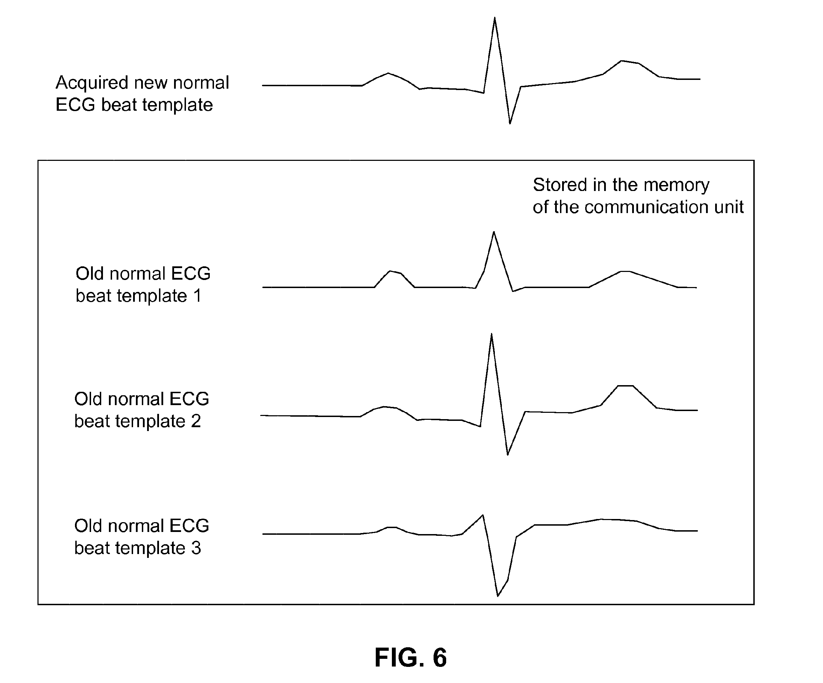

As illustrated in Figure 6, the portable communication unit 600 typically stores multiple (preferably at least 3) old normal ECG beat templates. Typically, these old normal ECG beat templates are first generated when the patient was seen by the physician or nurse. The physician or nurse can place the disposable unit 100 in multiple locations and/or orientations to select the preferred set of ECG leads that are believed to have good signal quality, and reveal important diagnostic features from the ECG waveform. For each preferred ECG lead position, a corresponding normal ECG beat template is retrieved from the electronic controller 200 and stored in the portable communication unit 600.

As described above, after changing the disposable unit 100, the patient can use the portable communication unit 600 to interrogate the electronic controller 200 to retrieve the newly acquired normal ECG beat template. In a preferred embodiment, the morphological similarity between the newly acquired normal ECG beat template and each of the old normal ECG beat templates that are stored in the portable communication unit 600, is respectively evaluated. One typical method to measure the morphological similarity is to calculate the correlation coefficient between the new and old normal ECG beat templates. Another method to quantify the morphological similarity is to calculate the adaptive signed correlation index between the new and old normal ECG beat templates, as disclosed in the U.S. Pat. Appl. 2009/0240300 Al assigned to the assignee of the present invention and incorporated herein by reference.

According to one typical embodiment, the newly acquired normal ECG beat template is considered acceptable only when its morphology is sufficiently similar to at least one of the old normal ECG beat templates stored in the portable communication unit 600. For example, the correlation coefficient must be greater than a predefined threshold, e.g. 0.85. The location and orientation of the disposable unit 100 can be considered to be similar to the ECG lead corresponding to the old normal ECG beat template that results in the highest morphological similarity, and this information could then be logged into the memory of the portable communication unit 600 and further communicated to the remote service center 700. For example, as illustrated in Figure 6, if the correlation coefficients between the newly acquired normal ECG beat template and each of the three old normal ECG beat templates are 0.70, 0.90, and 0.30, then the disposable unit 100 is believed to have similar lead location and orientation as the ECG lead that was used to generate the old normal ECG beat template 2.

According to another typical embodiment, the newly acquired normal ECG beat template is considered acceptable only when it has sufficiently high signal quality, for example, the signal to noise ratio (SNR) of the QRS complex in the ECG beat template is greater than a predefined threshold, e.g. 20dB. Other signal quality measures, such as the SNR of P wave or T wave, can also be evaluated based on the need of ECG interpretation.

Yet in another typical embodiment, the newly acquired normal ECG beat template is considered acceptable only when its morphology is sufficiently similar to at least one of the old normal ECG beat templates and it has sufficiently high signal quality as measured by the SNR.

On the other hand, if the newly acquired normal ECG beat template is considered not acceptable, for example because it has different morphology than the stored old normal ECG beat templates or its SNR is too low, then the patient is advised to reposition the disposable unit.

In one embodiment, the old normal ECG beat templates stored in the portable communication unit 600 are fixed after they are initially generated by the physician or nurse. Yet in another embodiment, the newly acquired normal ECG beat template - once it is considered acceptable (e.g. based on morphological analysis and signal quality assessment as described above) - can also be saved in the portable communication unit 600 and becomes (or even update) an old normal ECG beat template, so that it can be used for comparison with later acquired new normal ECG beat templates. This dynamic update of the old normal ECG beat template is necessary when the normal ECG morphology changes over time, for example due to the progression of heart diseases.

Therefore, quantitative feedback, such as the morphological similarity between acquired ECG waveform and the pre-stored ECG templates as well as the signal-to-noise ratio, can be provided to the patient to guide the proper placement of the disposable unit 100 on the body's surface. By this means, the ECG lead location/orientation of the disposable units 100 during the long-term monitoring period will be consistent, or at least their changes are acceptable (in terms of signal quality and morphology) and are known to the physician or nurse who can then properly interpret the recorded ECG.

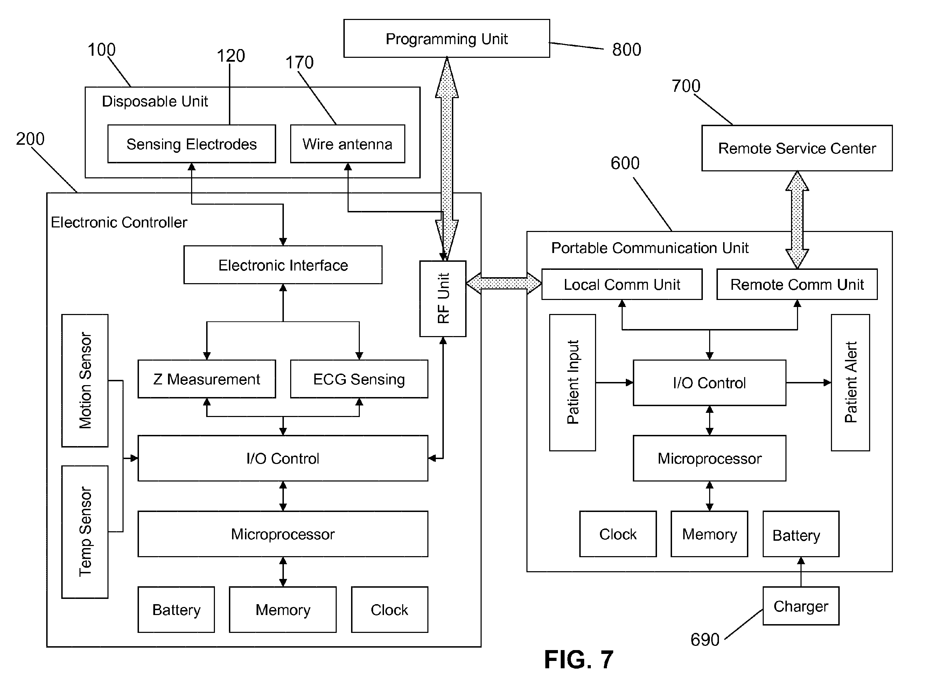

Figure 7 shows a block diagram of an electronic controller 200 connecting with a disposable unit 100 for cutaneous ECG monitoring and its interfaces with an external programming device 800 and a portable communication unit 600 which further communicates with the remote service center 700.

The electronic controller 200 comprises an electronic circuitry that is hermetically sealed inside a case. Enclosed inside the case, a microprocessor and associated circuitry make up the controller of the unit. The electronic controller 200 is powered by a battery which can last at least 1 year in normal operation without the need of recharging and maintains an internal clock for timing the operations. The microprocessor communicates with a memory via a bi-directional data bus. The memory typically comprises a ROM or RAM for program storage and a RAM for data storage.

By establishing the physical contact between the electrical conducting ports 210 on the case of the electronic controller 200 and the electrical conducting terminals 140 embedded in the disposable unit 100, the sensing electrodes 120 of the disposable unit 100 are electrically connected to an electronic interface of the electronic controller 200. The electronic interface preferably includes a feedthrough circuitry for noise reduction, a high voltage protection circuitry, a switch network circuitry for sensing channel selection, and front-end analog filters, as well-known in the art. The configurations of the interface circuitry (e.g. filter settings, sensing channel selection, etc.) can be programmed by the microprocessor.

In addition, by establishing the physical contact between the antenna interface 220 on the case of the electronic controller 200 and the conducting port 180 of the wire antenna 170 embedded inside the disposable unit 100, the RF unit of the electronic controller 200 electrically connects with the wire antenna 170.

The microprocessor connects to an I/O control unit to manage the input and output of the electronic controller. One input signal is the cutaneous ECG picked up by the sensing electrodes. After pre-processed by the interface circuitry, the cutaneous ECG signal is further processed by the ECG sensing unit which usually comprises amplifiers, analog-to-digital converters, digital filters, etc. as known in the art.

Another input signal is the impedance (Z) signal measured between the sensing electrodes. By injecting a small constant current (e.g., 100 µA, preferably biphasic) between two electrodes while measuring the voltage difference between the same or different pair of electrodes, the impedance is calculated as the ratio between the measured voltage difference and the injecting current strength. The impedance signal provides useful information on the integrity of the sensing channel. For example, when the sensing electrodes are in proper contact with the patient's skin, the measured Z will remain in a stable range, typically from several hundred to a few thousand ohms, depending on the materials and surface area of the sensing electrodes, the frequency of the injecting current, etc. Larger than normal Z may indicate there is loose contact between the sensing electrodes and the patient's skin. Smaller than normal Z may indicate short-circuit between the sensing electrodes, for example due to patient sweating. In addition, the continuously measured impedance signal may be further processed by the microprocessor to extract other physiological status of the patient, such as the respiratory rate.

Other types of biological signals measured by specific sensors can also serve as input to the electronic controller. For example, an on-board accelerometer can serve as a motion sensor that provides patient's activity signal to the electronic controller, an on-board temperature sensor can provide the cutaneous temperature signal to the electronic controller. Other types of input signals include, but are not limited to, the cutaneous oxygen saturation signal measured by an optical sensor, the heart sound signal measured by an acoustic sensor, etc.

By running the program stored in the memory, the microprocessor also sends instructions to the ECG sensing unit, the impedance measurement unit, and other input measurement units to control how these signals are acquired (e.g., gain, offset, filter settings, sampling frequency, sampling resolution, etc.).

The acquired biological signals are then stored in the device memory according to predefined storage modes. One typical mode is the queue-loop mode, meaning the acquired signals are stored in a predefined memory space, and while the allocated memory space is full, the newly acquired signals replace the oldest stored data. Another typical mode is the snapshot mode, meaning the acquired signals are stored in a predefined memory space, and while the allocated memory space is full, the newly acquired signals are not stored until the microprocessor decides to store another episode of data. Yet another typical mode is the mixed mode, in which one or more segments of allocated memory space store the acquired signals in queue-loop mode, whereas one or more segments of separately allocated memory space store the data in snapshot mode.

The acquired biological signals are analyzed by the microprocessor by running programmed algorithms. For example, the microprocessor continuously analyzes the acquired cutaneous ECG signals to detect the peak of QRS complex. Accordingly, the electronic controller measures the intervals between any two adjacent peaks of the detected QRS complexes, and these intervals are termed RR intervals. These measured RR intervals are stored in the device memory. Similarly, the microprocessor can also continuously analyze the acquired cutaneous ECG signals to measure other metrics of the QRS complex, such as the width of the QRS complex, the positive or negative peak amplitude of the QRS complex, the absolute area under the QRS complex, the maximum positive or negative slopes of the QRS complex, the dominant frequency component of the QRS complex, the complexity measures of the QRS complex, and so on. Likewise, the time series of these measured metrics are stored in the device memory for further analysis.

The electronic controller also includes a radio-frequency (RF) telemetry unit. The RF telemetry unit may be of the type well-known in the art for conveying various information, which it obtains from the electronic controller, to the external programmer, or for receiving programming parameters from the external programmer and then conveying it to the electronic controller. In one typical embodiment, the external programmer can interrogate the electronic controller and get its working status (e.g., battery status, sensing channel impedance, etc.) or the data recorded by the electronic controller (e.g. peak amplitude of the QRS complexes, statistics of measured RR intervals, etc.). In another typical embodiment, the external programmer can be used to activate or deactivate selected algorithms or update programmable parameters of the electronic controller.

In addition, the external portable communication unit to be described hereinafter can also communicate bi-directionally with the electronic controller through the telemetry unit. Preferably, the data that may be received from or sent to the external portable communication unit are more limited compared to the data that may be received from or sent to the external programmer.

In a preferred embodiment, the data that are transmitted from the external portable communication unit to the electronic controller are simple commands, such as trigger a snapshot of the acquired cutaneous ECG, retrieve most recently diagnostic information from the electronic controller, etc. These commands set the electronic controller into one of a number of modalities, wherein each modality is determined and controlled by parameters that can only be selected by a physician operating the external programmer using secure password or codes.

The data that are transmitted from the electronic controller to the external portable communication unit preferably include simple acknowledgment to confirm receiving the commands from the external portable communication unit, the signals warning the detection of abnormal conditions, such as detection of atrial fibrillation (AF), detection of high or low ventricular rate, detection of abnormal sensing impedance, detection of abnormal temperature, and so on. Other diagnostic information, such as the AF burden, the frequency of ectopic beats, snapshots of RR intervals or cutaneous ECG, etc. can also be transmitted to the external portable communication unit. Preferably, a physician operating the external programmer using secure password or codes, controls the enable or disable condition as well as the amount of data that can be transmitted from the electronic controller to the external portable communication unit.

Still refer to Figure 7. The external portable communication unit has a power source, such as a lithium battery, which provides power to the electrical components of the unit. The battery is chargeable by connecting to an external charger. The external portable communication unit also maintains an internal clock for timing its operations. The overall functioning of the external portable communication unit is controlled by its microprocessor, which reads and performs instructions stored in its associated memory. The instructions stored in memory preferably include instructions defining a communication protocol compatible with the electronic controller, and instructions defining a communication protocol compatible with the remote service center.

The microprocessor of the external portal communication unit communicates with an I/O control unit to read from the keypad (or press switches) the patient input commands. In an exemplary embodiment, one subset of the input commands is designed to configure the external portable communication unit, for example to turn on or off certain outputs as described hereinafter, or select specific communication protocols. Another subset of the input commands is designed to establish communication between the external portable communication unit and the remote service center through remote communication unit. For example, patient's input can command the external portable communication unit to transmit diagnostic information (retrieved from the electronic controller) to the remote service center and wait to receive acknowledgment. The third subset of the commands is designed to establish communication between the external portable communication unit and the electronic controller through local communication unit. For example, patient's input can command the external portable communication unit to transmit corresponding signals to the electronic controller to trigger recording a snapshot of the cutaneous ECG, to retrieve diagnostic information from the electronic controller, etc. The local communication unit also receives the acknowledgment and related diagnostic information sent from the electronic controller and conveys these data to the microprocessor for storage in the memory.

According to one exemplary embodiment of the present invention, upon receiving a predefined warning signal from the electronic controller (e.g., detection of AF, detection of high or low ventricular rate, detection of abnormal sensing impedance, detection of abnormal temperature, etc.), the microprocessor of the external portable communication unit communicates with the I/O control unit to generate output that is perceptible by the patient. Such output can be in the form of visible message, such as the light-up or blinking of a light emitting diode (LED) or the text message displayed in a liquid crystal display (LCD), or in the form of audible message, such as beep, ringing tone or pre-recorded voice messages played by a speaker or in the form of discernible vibration by a vibrator. According to the patient's preference, one or multiple types of warning message can be respectively turned on or off. For example, the visible warning message can be turned on while the audible warning message can be turned off during the night if the patient chooses not to be disturbed during sleep, even if the electronic controller detects AF. Besides generating warning messages, some diagnostic information that is received from the electronic controller and stored in memory (e.g., the heart rate) can also be provided to the patient in the form of visual or audible messages.

The external portable communication unit, via its remote communication unit, can further communicate with the remote service center. Such long-range communication apparatus can be in the form of a mobile radio network or a fixed-line telecommunication network or the internet, as well known in the art. Examples of such long-range communication apparatus have been taught in U.S. Pat. No. 6,470,215, U.S. Pat. No. 6,574,509, U.S. Pat. No. 6,622,043, all are assigned to the assignee of the present invention and incorporated herein by reference.

In one typical embodiment, the external portable communication unit transmits the electronic controller status information (e.g. battery status, sensing impedance, etc.) as well as relevant diagnostic information (e.g. AF burden, ectopic beat frequency, etc.) to the remote service center according to a predefined transmission frequency and schedule (e.g. every midnight, etc.). Yet in another typical embodiment, the external portable communication unit communicates with the remote service center in a trigger mode, for example upon receiving a warning signal from the electronic controller or upon receiving the patient trigger or when the filled memory buffer of the electronic controller reaches a predefined percentage of its capacity. In such cases, the external portable communication unit transmits critical diagnostic information stored in device memory (e.g. AF burden, mean heart rate, the ECG snapshot, etc.) to the remote service center.

The remote service center receives the information via compatible communication protocols, then sends acknowledgment back to the portable communication unit which may generate visible or audible output indicating receipt of the acknowledgment. The data received by the remote service center is stored in a secured central database and is promptly presented to the patient's physician or responsible expert through proper means, such as fax, email and text message, as known in the art. By reviewing the received diagnostic information, the physician can evaluate the patient's condition and provide expert advice to the patient who wishes to contact the physician before taking any action in response to the warning signals generated by the external portable communication unit.

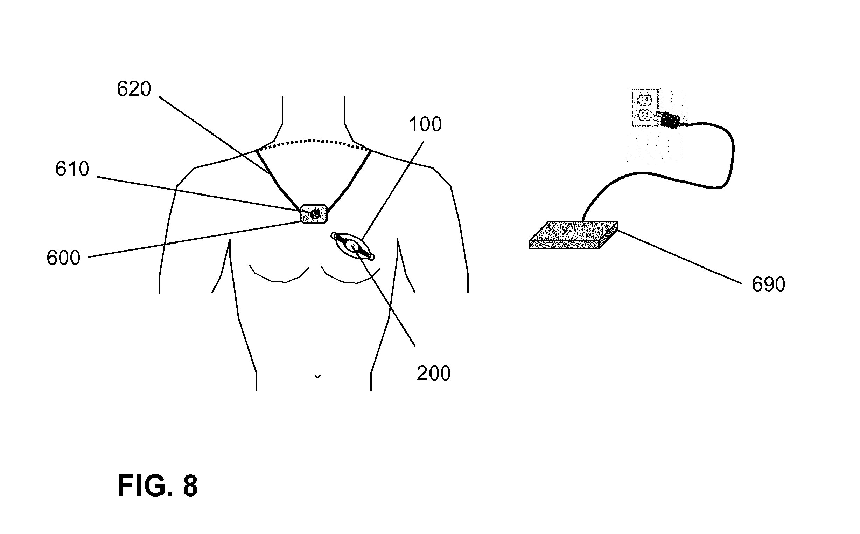

The external portable communication unit is designed to be easily carried by the patient. For example, it can be carried in the pocket or clipped on the belt or worn like a watch or a necklace, etc. As a schematic illustration, Figure 8 shows the external portable communication unit 600 having a loop antenna 620 designed in the form of a necklace. The figure also shows that the patient is wearing a disposable unit 100 which encloses an electronic controller 200 for cutaneous cardiac monitoring. The long antenna length encircles a loop area which forms a large effective aperture. In addition, the external portable communication unit 600 is close to the electronic controller 200 and their spatial distance and orientation are relatively stable. All these factors favor more reliable communications between the external portable communication unit 600 and the electronic controller 200.

Figure 8 also shows that the external portable communication unit 600 has a button 610 which can be pressed by the patient to trigger an event recording by the electronic controller 200. The recorded event, along with related diagnostic information stored by the electronic controller 200, are first transmitted to the external portable communication unit 600, which then relays the information to the remote service center through the wired or wireless Home Monitoring network.

The portable communication unit 600 can be recharged on a charging station 690. As known in the art, the charging can be implemented in either conductive charging (i.e. direct electrical contact between the battery and the charger) or inductive charging (i.e. use the electromagnetic field to transfer energy between the battery and the charger). During charging, the portable communication unit 600 can still communicate with the electronic controller 200, as long as their distance is within specified range, e.g. 6 feet. Preferably, the portable communication unit 600 can be powered by a backup battery, while its rechargeable battery is recharged in the charging station 690. Hence, patient can still wear the fully powered portable communication unit 600 while its rechargeable battery is recharged. Multiple mechanisms can be designed to improve the patient appliance for recharging the battery. For example, when the portable communication unit 600 detects its battery voltage is below a predefined value, a warning signal in various forms, such as audible beeps, vibrations, etc. is generated to alert the patient. In another example, the charging station 690 has a user-programmable unit that allows patient to set clock alarm (e.g. every night at 9 p.m.) as a reminder for charging the battery.

The disclosed system and apparatus provide a novel solution for long-term non-invasive cardiac monitoring. Patient can replace the disposable units as often as needed with the same electronic controller and the portable communication units that can operate for at least 1 year. No battery recharging is necessary for the electronic controller, thus causing little disruption to the cardiac monitoring. Besides conventional ECG monitoring capabilities, the automatic alert features (e.g. detection of skin-electrode contact and signal quality) can greatly improve the patient compliance which is essential for long-term monitoring. In conjunction with the Home Monitoring feature, this system minimizes the risk of memory saturation which is a common problem for most existing cardiac monitors. Moreover, quantitative feedback is provided to the patient on the quality of the acquired ECG signal to guide the proper replacement of the disposable units.

Further advantages such as potentially low cost of the system, the convenience of its usage, combined with the high diagnostic yield, represent high added value.

Although an exemplary embodiment of the present invention has been shown and described, it should be apparent to those of ordinary skill that a number of changes and modifications to the invention may be made without departing from the spirit and scope of the invention. All such changes, modifications and alterations should therefore be recognized as falling within the scope of the present invention.

## Claims

1. An apparatus for long-term cutaneous cardiac monitoring: comprising:
- a disposable unit (100) configured to be adhered to a patient's skin,
- an electronic controller (200) detachably connected to said disposable unit, wherein said disposable unit (100) comprises
- a substantially flat and flexible skin-contacting part that has at least two pick-up electrode poles (120) arranged to electrically contact a patient's skin at at least two spaced-apart locations,
- a wire antenna; (170) and
- a watertight chamber (150) attached to said flat and flexible skin contacting part and being configured to accommodate said electronic controller (200) in a watertight sealed manner and being able to open and close so as to allow removal and reinsertion of said electronic controller, on the inside of said chamber, electrical terminals (140) are provided that are electrically connected to said at least two electrode poles (120) and that are arranged to be operatively connected to respective contact ports (210) of said electronic controller (200), on the inside of said chamber, further a conducting antenna port (180) is provided that is electrically connected to said wire antenna (170) and that is arranged to be operatively connected to an antenna port (220) of said electronic controller (200); and
wherein said electronic controller (200) comprises
- a battery with a capacity sufficient to allow the electronic controller to operate for at least one year and
- electrical conducting ports arranged to make contact to said terminals and said conducting antenna port within said chamber and to thus establish electrical contact with the said at least two electrode poles and said wire antenna, respectively,
**characterised in that** the electronic controller (200) is configured
- to acquire, process and store the physiological signals to be picked up via said at least two electrode poles (120).

2. The apparatus of claim 1, **characterised in that** the electronic controller (200) is further configured to provide quantitative feedback on signal quality of a signal acquired via said at least two pick-up electrode poles (120).

3. The apparatus of claim 1 or 2, **characterised in that** the electronic controller (200) is further configured to maintain at least one normal ECG beat template.

4. The apparatus of claim 3, **characterised in that** the electronic controller (200) is further configured to construct said normal ECG beat template by averaging multiple normal beats of ECG signals acquired via said pick-up electrode poles and being aligned with a predefined fiducial point.

5. The apparatus of claim 3 or 4, **characterised in that** the electronic controller (200) is further configured to start constructing the normal ECG beat template immediately upon affixing said disposable unit on a patient's skin and to continuously update the normal ECG beat template based on acquired ECG signals.

6. The apparatus of claim 1, **characterised in that** the disposable unit (100) has three built-in electrode poles.

7. The apparatus of claim 1, **characterised in that** the electrode poles (120) are spaced apart by an inter-electrode distance of at least 3 cm.

8. The apparatus of claim 1, **characterised in that** the electronic controller (200) comprises an impedance measuring unit that is connected to said conducting ports for connecting said terminals, said impedance measuring unit being configured to measure an impedance signal between said at least two electrode poles.

9. A system for long-term cutaneous cardiac monitoring, comprising:
(1) a disposable unit (100) that has at least two built-in electrodes (120), a built-in wire antenna (170), a waterproof chamber (150) that can be opened and closed and an adhesive surface (110);
(2) an electronic controller (200) that has a battery life of at least 1 year and can acquire, process and store the physiological signals and can be fitted into the said disposable unit (100) and establish the electrical contact with the said at least two built-in electrodes (120) ; and
(3) a portable communication unit (600) that can wirelessly communicate bi-directionally with the said electronic controller (200) and further communicate bi-directionally with a remote service center (700).

10. The system of claim 9, **characterised in that** the portable communication unit (600) is configured to interrogate the electronic controller (200) to check the morphology and quality of an acquired ECG signal.

11. The system of claim 10, **characterised in that** the system is configured to wirelessly transmit a latest normal ECG beat template from the electronic controller (200) to the portable communication unit (600) and then compare a newly acquired normal ECG beat template to old normal ECG beat templates that are pre-stored in said portable communication unit (600).

12. The system of claim 9, **characterised in that** the portable communication unit (600) and/or the electronic controller (200) is configured to rate a newly acquired normal ECG beat template acceptable only when its morphology is sufficiently similar to at least one of said old normal ECG beat templates stored in the portable communication unit.

13. The system of claim 9, **characterised in that** the portable communication unit (600) and/or the electronic controller (200) is configured to rate a newly acquired normal ECG beat template acceptable only when it has sufficiently high signal quality, in particular if the signal-to-noise ratio of an QRS complex in said ECG beat template is greater than a predefined threshold.

14. The system of claim 9, **characterised in that** the portable communication unit (600) and/or the electronic controller (200) is configured to rate a newly acquired normal ECG beat template acceptable only when its morphology is sufficiently similar to at least one of the old normal ECG beat templates and it has sufficiently high signal quality as measured by the signal to noise ratio.

15. The system of one of claims 12 to 14, **characterised in that** the portable communication unit (600) and/or the electronic controller (200) is configured to generate a signal indicating a necessity to reposition said disposable unit if the newly acquired normal ECG beat template is rated not acceptable.
